# EUROPEAN PATENT APPLICATION

(11) **EP 3 153 200 A1**
(43) Date of publication of application: **12.04.2017**
(21) Application number: 16154240.2
(22) Date of filing: 04.02.2016
(51) Int. Cl.: A61M 16/04, A61B 1/005, A61B 1/01, A61B 1/00

(54) **FLEXIBLE BAR-SHAPED CAMERA AND INTUBATION DEVICE THEREOF**

(30) Priority: 05.10.2015 TW 104132743
(71) Applicant: Chen, Tien-Sheng, 112 Taipei City (TW)
(72) Inventor: Chen, Tien-Sheng, 112 Taipei City (TW)
(74) Representative: Altenburg, Bernardus Stephanus Franciscus

(57) **Abstract**

A flexible bar-shaped camera (1,1a) and an intubation device (100) thereof are disclosed. The flexible bar-shaped camera (1,1a) is applied to the intubation device (100) and placed inside an endotracheal tube (90). The intubation device (100) has a display screen (32). The flexible bar-shaped camera (1,1a) includes a main body (10). The endotracheal tube (90) has a tube front end (91) and a first curved end (92). The main body (10) has a camera end (11), a connecting end (12), and a second curved end (13), wherein the camera end (11) is located at the second curved end (13). After the flexible bar-shaped camera (1,1a) is placed inside the endotracheal tube (90), the second curved end (13) touches the tube front end (91) and the second curved end (13) situates in the first curved end (92) such that the camera end (11) is parallel to the display screen (32)

## Description

### BACKGROUND OF THE INVENTION

### Field of the Invention

The present invention relates a flexible bar-shaped camera and an intubation device using the same; more particularly, the present invention relates to a flexible bar-shaped camera and an intubation device using the same capable of utilizing a curved end of the flexible bar-shaped camera to achieve an object of camera orientation.

### Description of the Related Art

For patients whose breathing is suspended, endotracheal intubation is a necessary medical procedure for maintaining the breathing function. In order to keep the patient's vital signs, the medical staff has to place an endotracheal tube into the patient's trachea in a short amount of time so as to provide oxygen. Therefore, it has become an important task for the medical staff to rapidly and effectively perform intubation. Practically, the medical staff often utilizes an intubation device provided with a camera to confirm the location of the patient's trachea, and then places the endotracheal tube into the patient's trachea. Conventionally, a widely-used intubation device has a camera installed in a side edge of an endotracheal tube guide slot, so that after the medical staff observes the opening of the patient's trachea through the camera, the medical staff can then push the endotracheal tube located next to the side edge of the camera into the patient's trachea. However, due to differences of patients' body structures, the field of view of the camera might possibly be blocked by the endotracheal tube, which makes the medical staff incapable of precisely placing the endotracheal tube into the patient's trachea. Therefore, there is still a need to overcome the inconvenience while using a conventional intubation device.

In order to provide a more precise field of view for intubation, there is a better design of installing the camera inside the endotracheal tube. However, because the shape of the endotracheal tube is longitudinal, the camera also has to be longitudinal-shaped in order to be placed into the endotracheal tube; moreover, the camera has to be placed inside the endotracheal tube first, and then both parts are placed in an endotracheal tube guide slot of the intubation device for movement. As a result, this kind of camera not only needs to be placed inside the endotracheal tube, but also needs to move with the endotracheal tube along the endotracheal tube guide slot. Therefore, a tube body of this kind of camera has to be made of soft materials for facilitating the medical staff to operate. However, a soft tube body would easily cause an image-capturing direction of a camera lens of the longitudinal-shaped camera to be interfered by external force and thus resulting in a biased direction. In other words, the abovementioned design cannot guarantee that images captured by the camera lens of the longitudinal-shaped camera are all facing upwards and towards the patient's trachea.

Meanwhile, after the intubation device enters the patient's mouth, the medical staff has no control right of controlling the image-capturing direction of the camera lens. Instead, the medical staff can only accommodate with the images captured by the camera lens to perform intubation. However, because these images are not always facing upwards and towards the patient's trachea, it is difficult to perform intubation. Therefore, the intubation device having its camera placed inside the endotracheal tube is not very popular in the market.

Because endotracheal intubation is the key to keep the patient alive, there is a need to provide an intubation device capable of orientating the longitude-shaped camera, so as to provide a more convenient and precise endotracheal intubation procedure.

Therefore, the present invention provides a flexible bar-shaped camera and an intubation device using the same to mitigate and/or obviate the aforementioned problems.

### SUMMARY OF THE INVENTION

It is an object of the present invention to provide a flexible bar-shaped camera capable of utilizing a curved end of the flexible bar-shaped camera to achieve an object of camera orientation.

It is another object of the present invention to provide an intubation device using the abovementioned flexible bar-shaped camera.

To achieve the abovementioned objects, the present invention provides a flexible bar-shaped camera, which is applied to an intubation device. The intubation device includes a display screen. The flexible bar-shaped camera is placed inside an endotracheal tube. The endotracheal tube includes a tube front end and a first curved end. The flexible bar-shaped camera comprises a main body. The main body comprises a camera end, a connecting end and a second curved end. The camera end is located at the second curved end. After the flexible bar-shaped camera is placed inside the endotracheal tube, the second curved end touches the tube front end and the second curved end situates in the first curved end, such that the camera end is parallel to the display screen, and therefore images captured by the camera end are all facing upwards and towards a patent's trachea.

The present invention further provides an intubation device, which is used for placing an endotracheal tube into a patient's trachea. The endotracheal tube includes a tube front end and a first curved end. The intubation device comprises a flexible bar-shaped camera, a gripping portion and a guiding portion. The gripping portion includes a display screen. The flexible bar-shaped camera comprises a main body. The main body comprises a camera end, a connecting end and a second curved end. The camera end is located at the second curved end. The connecting end is connected to the gripping portion. After the flexible bar-shaped camera is placed inside the endotracheal tube, the second curved end touches the tube front end and the second curved end situates in the first curved end, such that the camera end is parallel to the display screen. The guiding portion is connected to the gripping portion. The guiding portion includes a front end and a rear end. The rear end of the guiding portion is connected to the gripping portion. The flexible bar-shaped camera and the endotracheal tube are movably installed in the guiding portion, such that images captured by the camera end are all facing upwards and towards the patient's trachea.

According to the design of matching the curvature of the second curved end with the curvature of the first curved end, an object of camera orientation can be achieved, thereby increasing the precision of endotracheal intubation.

Other objects, advantages, and novel features of the invention will become more apparent from the following detailed description when taken in conjunction with the accompanying drawings.

### BRIEF DESCRIPTION OF THE DRAWINGS

These and other objects and advantages of the present invention will become apparent from the following description of the accompanying drawings, which disclose several embodiments of the present invention. It is to be understood that the drawings are to be used for purposes of illustration only, and not as a definition of the invention.

In the drawings, wherein similar reference numerals denote similar elements throughout the several views:
FIG. 1 illustrates one schematic drawing of a flexible bar-shaped camera according to one embodiment of the present invention.
FIG. 2 illustrates a schematic drawing of the freely placed flexible bar-shaped camera according to one embodiment of the present invention.
FIG. 3 illustrates another schematic drawing of the flexible bar-shaped camera according to one embodiment of the present invention.
FIG. 4 illustrates a schematic drawing of the flexible bar-shaped camera moving within an endotracheal tube according to the present invention.
FIG. 5 illustrates a cross-sectional schematic drawing of the flexible bar-shaped camera placed inside the endotracheal tube.
FIG. 6 illustrates an exploded view of an intubation device according to one embodiment of the present invention.
FIG. 7 illustrates a schematic drawing of the intubation device according to one embodiment of the present invention.
FIG. 8 illustrates a cross-sectional schematic drawing of the intubation device according to one embodiment of the present invention.
FIG. 9 illustrates a schematic drawing showing display directions of the flexible bar-shaped camera and a display screen according to the present invention.

### DETAILED DESCRIPTION OF THE PREFERRED EMBODIMENT

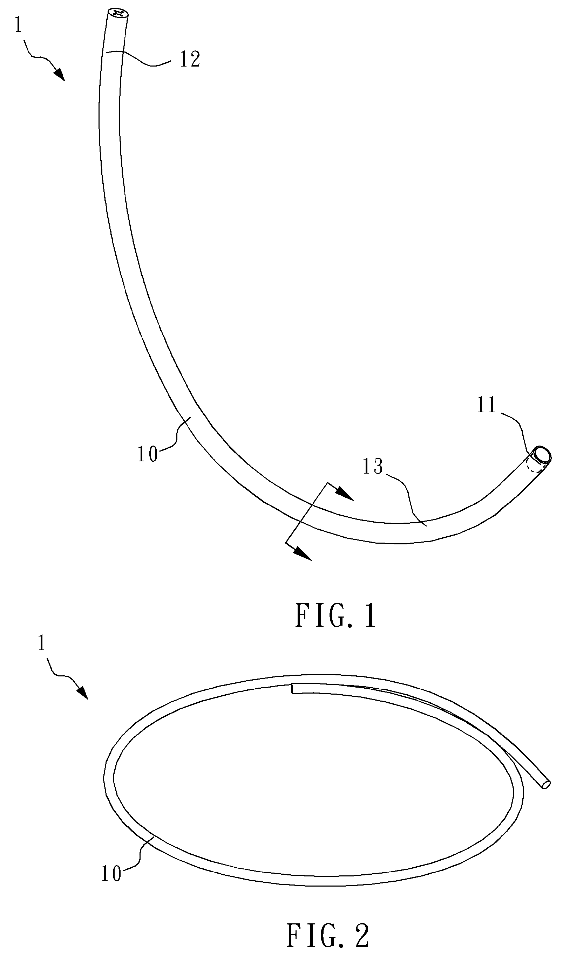

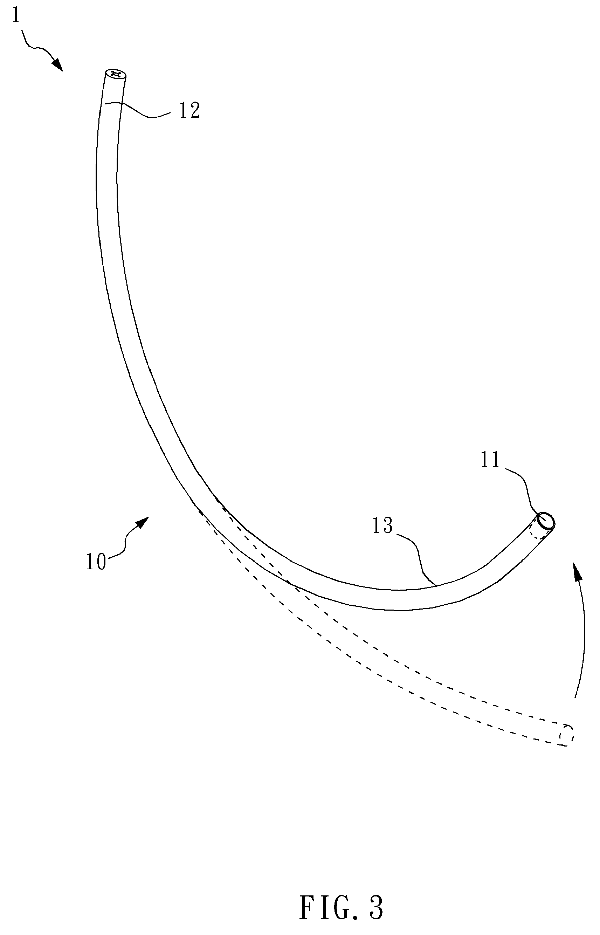

Please refer to FIG. 1 to FIG. 3. FIG. 1 illustrates one schematic drawing of a flexible bar-shaped camera according to one embodiment of the present invention; FIG. 2 illustrates a schematic drawing of the freely placed flexible bar-shaped camera according to one embodiment of the present invention; and FIG. 3 illustrates another schematic drawing of the flexible bar-shaped camera according to one embodiment of the present invention.

As shown in FIG. 1, the flexible bar-shaped camera 1 of the present invention comprises a main body 10. The main body 10 includes a camera end 11, a connecting end 12 and a second curved end 13. The camera end 11 is located at the second curved end 13, and the length of the second curved end 13 is approximately half or one-third of the length of the main body 10. According to another embodiment of the present invention, the main body 10 and the second curved end 13 can be directly made of thermoplastic elastomer. In one embodiment, the thermoplastic elastomer is a so-called TPE (thermal plastic elastomer) composite material, which can maintain the curvature of the second curved end 13 by means of heat setting. The heat setting procedure applied in this embodiment is to put a plastic cover member of the main body 10 in an environment at 100±20 degree Celsius (e.g. an oven) and rest for 10±3 minutes to complete the setting.

As shown in FIG. 2, when the flexible bar-shaped camera 1 is freely placed, the main body 10 naturally looks like in a circular shape. Further, as shown in FIG. 3, because the main body 10 is characteristic in bending itself naturally, the second curved end 13 can also recover to its original shape after deformation. Please note that according to one embodiment of the present invention, in order to accommodating patients of different figures, the length of the main body 10 is 50±10 centimeters, the arch length of the second curved end 13 is 40±10 centimeters, and the bending diameter of the second curved end 13 is 15±5 centimeters.

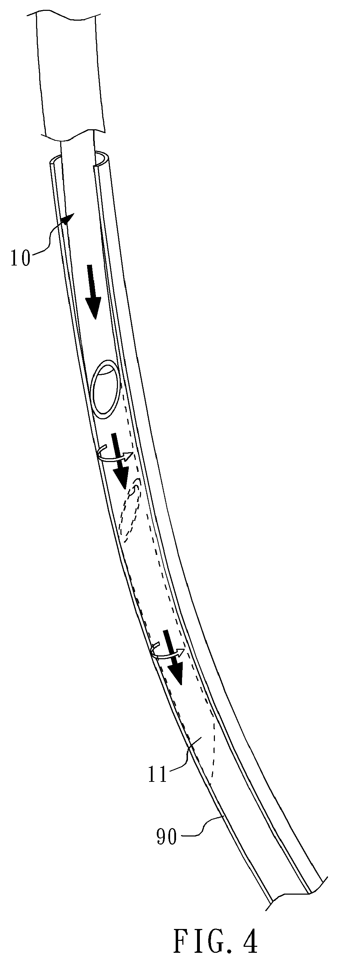

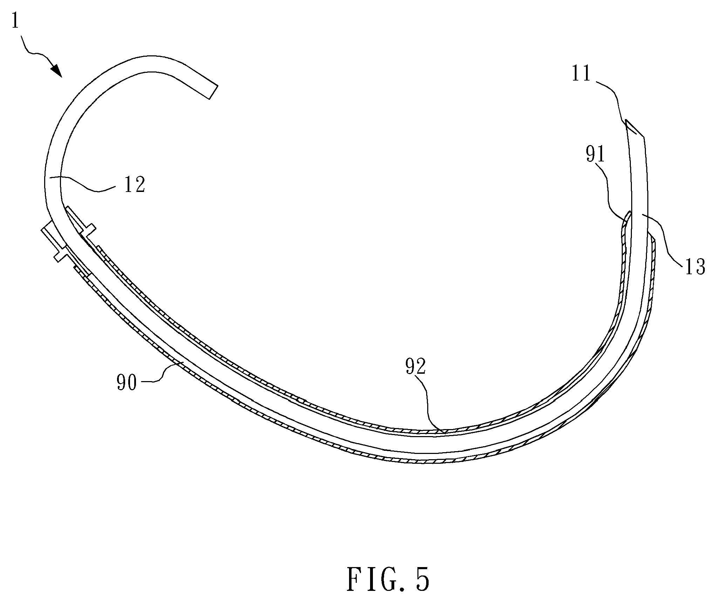

Please refer to FIG. 4 and FIG. 5. FIG. 4 illustrates a schematic drawing of the flexible bar-shaped camera moving within an endotracheal tube according to the present invention; and FIG. 5 illustrates a cross-sectional schematic drawing of the flexible bar-shaped camera placed inside the endotracheal tube.

As shown in FIG. 4 and FIG. 5, the flexible bar-shaped camera 1 is used along with an endotracheal tube 90. In use of the present invention, the flexible bar-shaped camera 1 has to be placed inside the endotracheal tube 90. The endotracheal tube 90 includes a tube front end 91 and a first curved end 92. Generally, according to sizes of tracheas of patents in different figures, the bending diameter of the first curved end 92 is approximately 30±10 centimeters. As shown in FIG. 4 and FIG. 5, after the flexible bar-shaped camera 1a is placed inside the endotracheal tube 90, because the endotracheal tube 90 and the main body 10 are both curved tubes, and the bending diameter of the second curved end 13 is smaller than the bending diameter of the first curved end 92 due to the influence of the bending diameter of the first curved end 92, the second curved end 13 would situate in an inner side edge of the first curved end 92, and the second curved end 13 touches the tube front end 91 so as to keep the camera end 11 facing towards the patient's trachea thereby achieving the object of orientating the camera end 11. In this embodiment, the bending diameter of the second curved end 13 is 15±5 centimeters.

Furthermore, as shown in FIG. 4 and FIG. 5, after the main body 10 is placed inside the endotracheal tube 90, the camera end 11 would rotate at least once because the curved shapes of the endotracheal tube 90 and the main body 10 interfere with each other, so that the camera end 11 can keep facing towards the patient's trachea and thus achieving the object of orientating the camera end 11, such that images captured by the camera end 11 are all facing upwards and towards the patient's trachea without receiving any inverse images. Please note the camera end 11 facing towards the patient's trachea means the camera end 11 always remaining parallel to a display screen of the intubation device while the medical staff using the intubation device to place the endotracheal tube 90 into the patient's trachea; or it means the images captured by the camera end 11 all facing upwards and towards the patient's trachea without resulting in any inverse images.

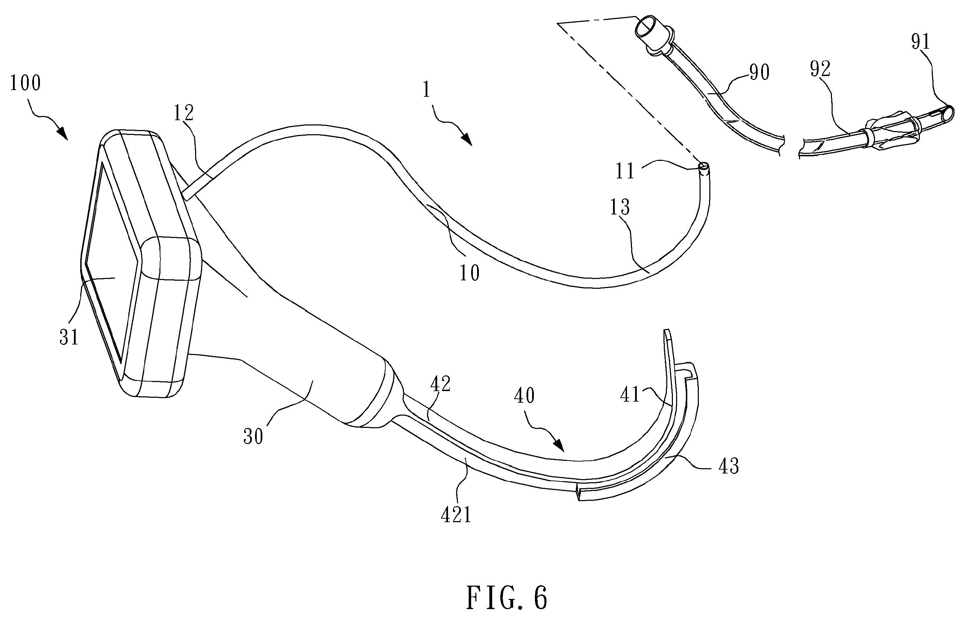

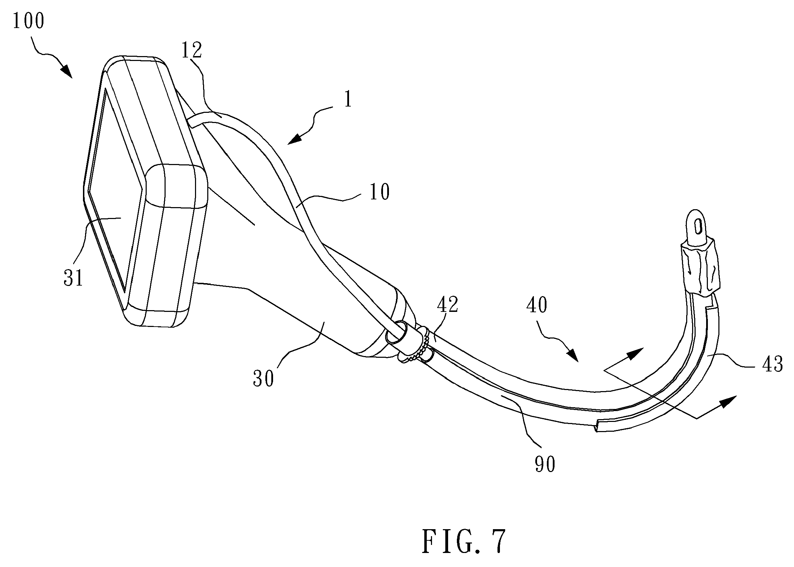

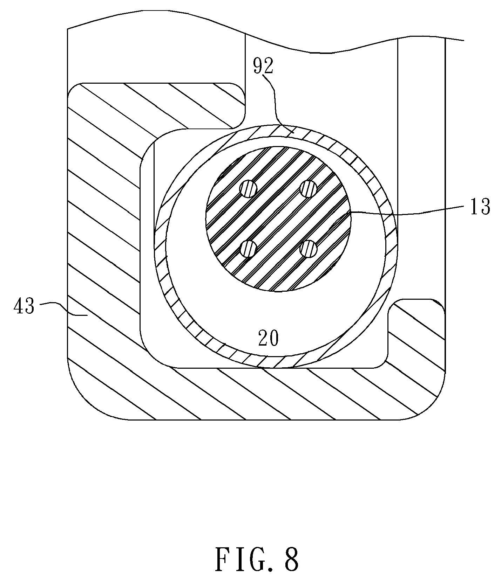

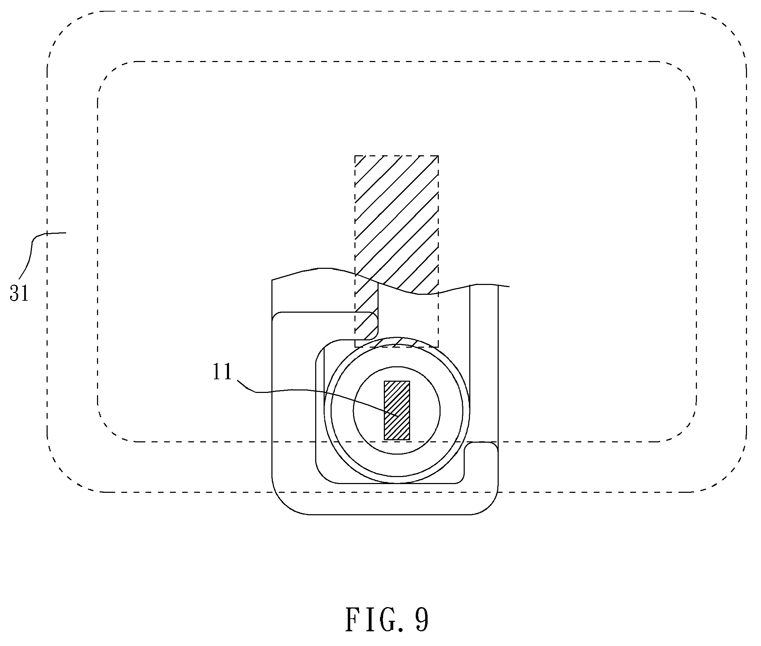

Please refer to FIG. 6 to FIG. 9. FIG. 6 illustrates an exploded view of an intubation device according to one embodiment of the present invention; FIG. 7 illustrates a schematic drawing of the intubation device according to one embodiment of the present invention; FIG. 8 illustrates a cross-sectional schematic drawing of the intubation device according to one embodiment of the present invention; and FIG. 9 illustrates a schematic drawing showing display directions of the flexible bar-shaped camera and a display screen according to the present invention.

The intubation device 100 of the present invention is used for assisting the medical technician or the medical staff to place the endotracheal tube 90 into the patient's trachea. The endotracheal tube 90 includes a tube front end 91 and a first curved end 92. According to one embodiment of the present invention, as shown in FIG. 6, the intubation device 100 of the present invention comprises a flexible bar-shaped camera 1, a gripping portion 30 and a guiding portion 40, wherein the flexible bar-shaped camera 1 is placed inside the endotracheal tube 90, and the guiding portion 40 is connected to the gripping portion 30.

As shown in FIG. 6 and FIG. 7, the gripping portion 30 includes a display screen 1, and the flexible bar-shaped camera 1 comprises a main body 10. The main body 10 includes a camera end 11, a connecting end 12 and a second curved end 13. The camera end 11 is located at the second curved end 13. The connecting end 12 is connected to the gripping portion 30. After the flexible bar-shaped camera 1 is placed inside the endotracheal tube 90, the second curved end 13 touches the tube front end 91, and the second curved end 13 situates in the first curved end 92, so as to achieve the object of always keeping the camera end 11 facing upwards and towards the patient's trachea as shown in FIG. 9, in order to make sure no inverse images would be captured during the endotracheal intubation procedure. According to one embodiment of the present invention, the length of the main body 10 is 50±10 centimeters, and the arch length of the second curved end 13 is 40±10 centimeters. By means of heat setting, the curvature of the second curved end 13 of the flexible bar-shaped camera 1 can be maintained.

The guiding portion 40 includes a front end 41 and a rear end 42. The rear end 42 of the guiding portion 40 is connected to the gripping portion 30. The flexible bar-shaped camera 1 and the endotracheal tube 90 are movably installed in the guiding portion 40 for facilitating the placement of the endotracheal tube 90 into the patient's trachea. According to one embodiment of the present invention, the guiding portion 40 further comprises a guide slot 43. The guide slot 43 is used for placing the endotracheal tube 90 and the flexible bar-shaped camera 1. The guide slot 43 is provided in the front end 41 of the guiding portion 40. A passage 421 is formed in the rear end 42 of the guiding portion 40, so as to place the endotracheal tube 90 and the flexible bar-shaped camera 1 in the guide slot 43, and thus forming the cross-sectional schematic drawing as shown in FIG. 8.

As shown in FIG. 8, after the flexible bar-shaped camera 1 is placed inside the endotracheal tube 90, because the bending diameter of the second curved end 13 is smaller than the bending diameter of the first curved end 92, the second curved end 13 would situate in the inner side edge of the first curved end 92. That is, according to FIG. 8, the flexible bar-shaped camera 1 is located close to the top of the endotracheal tube 90 instead of being located in the center of the endotracheal tube 90. Therefore, as shown in FIG. 9, an image-capturing direction of the camera end 11 can always be parallel to the display screen 31 of the intubation device 100 due to the shape limitation of the first curved end 92 and the second curved end 13, thereby achieving the object of orientating the camera end 11. That is, the first curved end 92 retains the image-capturing direction of the camera end 11. After the flexible bar-shaped camera 1 is placed inside the endotracheal tube 90 and is placed in the guide slot 43, because of the mechanism limitation caused by the curvature of the guide slot 43, the endotracheal tube 90 and the flexible bar-shaped camera 1 can be orientated at the same time, therefore the present invention can make sure the image-capturing direction of the camera end 11 is always parallel to the display screen 31 of the intubation device 100.

According to the design of the bending diameter of the second curved end 13 being smaller than the bending diameter of the first curved end 92, the present invention achieves the object of orientating the camera end 11 (to be parallel to the display screen 31), so as to provide the medical staff with a correct field of view. That is, the viewing angle of the camera end 11 always faces upwards and towards the patient's trachea during the endotracheal intubation procedure without resulting in any inverse images, thereby increasing the precision and success rate of endotracheal intubation.

Although the present invention has been explained in relation to its preferred embodiments, it is to be understood that many other possible modifications and variations can be made without departing from the spirit and scope of the invention as hereinafter claimed.

## Claims

1. A flexible bar-shaped camera (1,1a), applied to an intubation device (100), the intubation device (100) including a display screen (31), the flexible bar-shaped camera (1,1a) being placed inside an endotracheal tube (90), and the endotracheal tube (90) including a tube front end (91) and a first curved end (92), the flexible bar-shaped camera (1,1a) comprising:
a main body (10), comprising a camera end (11), a connecting end (12) and a second curved end (13), wherein the camera end (11) is located at the second curved end (13), and after the flexible bar-shaped camera (1,1a) is placed inside the endotracheal tube (90), the second curved end (13) touches the tube front end (91) and the second curved end (13) situates in the first curved end (92), such that the camera end (11) is parallel to the display screen (31).

2. The flexible bar-shaped camera (1,1a) as claimed in claim 1, wherein the length of the main body (10) is 50±10 centimeters, and the arc length of the second curved end (13) is 40±10 centimeters.

3. The flexible bar-shaped camera (1,1a) as claimed in claim 1 or 2, wherein the bending diameter of the second curved end (13) is 15±5 centimeters.

4. An intubation device (100), used for placing an endotracheal tube (90) into a patient's trachea, wherein the endotracheal tube (90) includes a tube front end (91) and a first curved end (92), the intubation device (100) comprising:
a gripping portion (30), including a display screen (31); and
a flexible bar-shaped camera (1,1a) as claimed in any of claim 1 to claim 3.

5. The intubation device (100) as claimed in claim 4, further comprising a guiding portion (40) including a guide slot (43) used for placing the endotracheal tube (90) and the flexible bar-shaped camera (1,1a), wherein the guide slot (43) is provided in a front end of the guiding portion, and a passage is formed in a rear end of the guiding portion (40), so as to place the endotracheal tube (90) and the flexible bar-shaped camera (1,1a) in the guide slot (43).
